# EUROPEAN PATENT SPECIFICATION

(11) **EP 4 208 151 B1**
(45) Date of publication and mention of the grant of the patent: **10.07.2024**
(21) Application number: 21763144.9
(22) Date of filing: 31.08.2021
(51) Int. Cl.: A61K 9/00, A61K 9/14, A61K 9/19, A61K 31/444, A61K 31/4545, A61K 31/465, A61K 47/18, A61K 47/26, A61M 15/06, A61P 29/00

(54) **FREEZE DRIED LOW HYGROSCOPICITY ACTIVE POWDER COMPOSITIONS**
GEFRIERGETROCKNETE AKTIVE PULVERZUSAMMENSETZUNGEN MIT NIEDRIGER HYGROSKOPIZITÄT
COMPOSITIONS DE POUDRE ACTIVE LYOPHILISÉE À FAIBLE HYGROSCOPICITÉ

(30) Priority: 03.09.2020 EP 20194435
(43) Date of publication of application: 12.07.2023
(73) Proprietor: Philip Morris Products S.A., 2000 Neuchâtel (CH)
(72) Inventor: SPADARO, Fabiana, 2000 Neuchâtel (CH); ZUBER, Gerard, 2000 Neuchâtel (CH)
(74) Representative: Parsi Mendiola, Joshua
(86) International application number: PCT/IB2021/057951
(87) International publication number: WO 2022/049486

(56) References cited:
- WO-A1-2018/163085
- WO-A1-2020/023614
- WO-A1-2020/127225
- US-A1- 2019 133 940
- KAIALY WASEEM ET AL: "Freeze-Dried Mannitol for Superior Pulmonary Drug Delivery via Dry Powder Inhaler", PHARMACEUTICAL RESEARCH, vol. 30, no. 2, 16 October 2012 (2012-10-16), US, pages 458 - 477, XP055776852, ISSN: 0724-8741, DOI: 10.1007/s11095-012-0892-4

## Description

The present disclosure relates to freeze drying active powder compositions that exhibit low hygroscopicity. Sugar alcohol and an alkaloid are freeze dried and then milled to form crystalline dry particles.

Active powder compositions have been described having an amorphous structure. An amorphous structure lacks long range order characteristics of a crystalline form. These amorphous powder compositions generally exhibit a greater water solubility than crystalline counterpart powder compositions and may exhibit a reduced shelf life or delivery due to ambient moisture absorption.

Amorphous powder compositions tend to be hygroscopic and agglomerate, become sticky, or even liquify when exposed to humid conditions, for example, a relative humidity at or above about 40% and temperatures at or above about 25°C. Tropical conditions, for example, a relative humidity at or above about 60% and temperatures at or above about 30°C may exacerbate these problems. Transport and storage of these amorphous powder compositions may require hermetic packaging to maintain the stability of the amorphous powder compositions. Once the hermetic seal has been compromised (for example, package has been opened by a consumer), the contents may rapidly absorb humidity. In addition, delivery of amorphous powder compositions to the lungs of the consumer may require separation of humidity from the inhalation airflow, resulting in increased complexity and cost of the associated inhaler device or article.

WO 2018/163085 A1 discloses a method of forming an inhalable powder composition comprising combining lactose and leucine with nicotine tartrate and a liquid carrier to form a liquid mixture, drying the liquid mixture to form a dry composition, and milling the composition to form dry powder particles having a particle size of less than 5 microns.

It would be desirable to provide a freeze dried active powder composition that exhibits a lowered hygroscopicity. It would be desirable to provide a freeze dried active powder composition that resists water or humidity absorption when exposed to humid or tropical conditions. It would be desirable to provide a freeze dried active powder composition that exhibits a reduced agglomeration when exposed to humid or tropical conditions. It would be desirable to provide a freeze dried active powder composition that is stable and possesses a long shelf life.

According to the present invention, there is provided a method of forming an inhalable powder composition comprising combining a sugar alcohol with an alkaloid and a liquid carrier to form a liquid mixture, the sugar alcohol comprising erythritol, myo-inositol, adonitol, mannitol, and xylitol, or a combination thereof; freeze drying the liquid mixture to form a crystalline dry composition, and milling the crystalline dry composition to form crystalline dry powder particles having a mass median aerodynamic diameter (MMAD) of about 5 micrometres or less when measured with a cascade impactor.

Advantageously, the inhalable powder exhibits a low or reduced hygroscopicity. The inhalable powder does not readily absorb water or moisture when exposed to humid or tropical conditions. The inhalable powder does not readily agglomerate when exposed to humid or tropical conditions. Further advantageously, the inhalable powder is stable and possesses a long shelf life.

The term "freeze drying" refers to a low temperature dehydration process that involves freezing the product, lowering the pressure, and removing frozen solvent or frozen liquid carrier by sublimation.

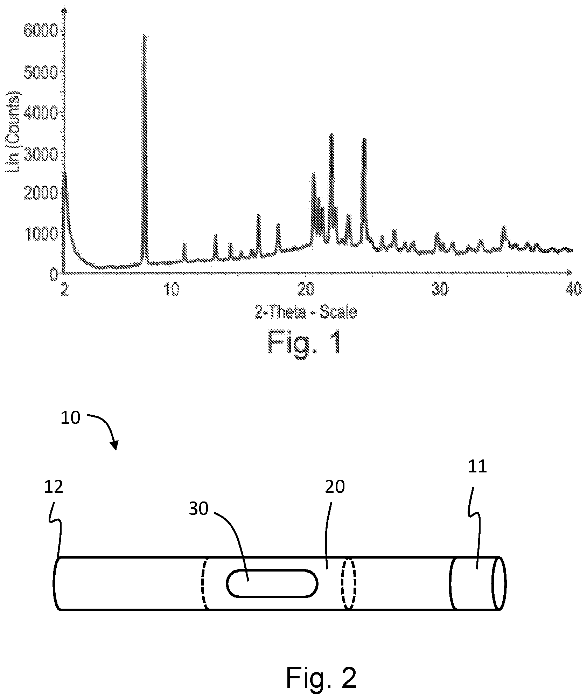

The term "crystalline" refers to a solid form of a compound exhibiting long range order in a microscopic lattice. A skilled person can identify a crystalline material by inspection of an X-ray diffraction (XRD) pattern of that material, such as a powder X-ray diffraction (pXRD) trace. For example, a crystalline XRD pattern is illustrated in FIG. 1. A crystalline material may exhibit some amorphous structure.

The term "active agent" refers to an alkaloid or other pharmaceutically active ingredient. The "active agent" always refers to just the active agent component of a compound. Preferably the active agent may be a salt of the active agent.

The term "low hygroscopicity" refers to a measured hygroscopicity value of a water uptake of 5% wt or less, or 4% or less, or 3% or less, or 2.5% or less, or 2% or less. Hygroscopicity may be determined by measuring the water uptake or absorption (wt% increase) of the sample when exposed to tropical conditions (75% relative humidity and 30 degrees Celsius atmosphere) for a 20-minute time duration. Prior to exposure to tropical conditions, the sample is equilibrated at 0% relative humidity, and 25 degrees Celsius atmosphere for 24 hours to reduce or remove non-bonded water from the sample. The hygroscopicity value is measured utilizing a "Vapor Sorption Analyzer SPSx-1u High Load" manufactured by ProUmid GmbH & Co. KG, as described in the Examples below.

The method may include mixing the alkaloid with the sugar alcohol and the liquid carrier, where the alkaloid is added in a solid form. In some embodiments, the alkaloid is a base and is mixed with an acid to form a salt. For example, the alkaloid may comprise nicotine or anatabine or anabasine provided as a free base and combined with an acid to form a pharmaceutically acceptable salt.

Any pharmaceutically acceptable salt may be used to form the salt of the alkaloid. In a preferred embodiment, the salt of the alkaloid may be solid at room temperature (for example, solid at 25 °C). Suitable salts include, for example, a salt of aspartic acid ("aspartate"), gentisic acid ("gentisate"), benzoic acid ("benzoate"), lactic acid ("lactate"), malic acid ("malate"), fumaric acid ("fumarate"), maleic acid ("maleate"), muconic acid ("muconate"), hydrochloric acid ("hydrochlorate"), alfa-resorcylic acid ("alfa-resorcylate"), beta-resorcylic acid ("beta-resorcylate"), oxalic acid ("oxalate"), p-anisic acid ("anisate"), tartaric acid ("bitartrate"), or glutaric acid ("glutarate"). Preferably the salt comprises aspartate, bitartrate, malate, or glutarate.

A preferred alkaloid is nicotine. Nicotine may preferably be a nicotine salt. Nicotine salts include, for example, nicotine aspartate, nicotine bitartrate, nicotine glutarate, nicotine lactate, nicotine gentisate, nicotine benzoate, nicotine fumarate, nicotine hydrochlorate, nicotine alfa-resorcylate, nicotine beta-resorcylate, nicotine oxalate, nicotine malate, and nicotine anisate. A preferred nicotine salt is selected from one or more of nicotine bitartrate, nicotine aspartate, nicotine malate, and nicotine glutarate.

Another preferred alkaloid is anatabine. Anatabine may preferably be an anatabine salt. A preferred anatabine salt is anatabine glutarate. Anatabine glutarate is described, for example, in WO2020/127225A1.

Anatabine is an alkaloid present in tobacco and, in lower concentrations, in a variety of foods, including green tomatoes, green potatoes, ripe red peppers, tomatillos, and sundried tomatoes. It is a main active component of the marketed dietary supplement anatabloc providing anti-inflammatory support, as disclosed in US 9,387,201 and WO 2013/032558. The preparation of isolated forms of anatabine is described in WO 2011/119722, for example.

Anatabine is also known as 3-(1,2,3,6-tetrahydropyridin-2-yl)pyridine. Enantioselective syntheses of S- and R-enantiomers of anatabine are described, for example, in Ayers, J. T.; Xu, R.; Dwoskin, L. P.; Crooks, P. A. A general procedure for the enantioselective synthesis of the minor Tobacco alkaloids nornicotine, anabasine, and anatabine. The AAPS Journal 2005; 7(3) Article 75.

The term "anatabine" as used here may refer to (1) a racemic mixture of anatabine (R,S); (2) a purified form of S-(-)-anatabine; or (3) a purified form of R-(+)-anatabine. A preferred anatabine compound is anatabine salt such as anatabine glutarate or 3-(1,2,3,6-tetrahydropyridin-2-yl)pyridine glutarate. Preferably, the 3-(1,2,3,6-tetrahydropyridin-2-yl)pyridine glutarate has a 1:1 molar ratio of 3-(1,2,3,6-tetrahydropyridin-2-yl)pyridine to glutarate.

Pharmaceutically acceptable salts of anatabine are described in US Pat. 8,207,346 and US Pat. 8,557,999. In particular, Example 6 of US Pat. 8,207,346 and Example 6 of US Pat. 8,557,999 describe the preparation of anatabine tartrate and anatabine citrate by addition of tartaric acid or citric acid to a solution of anatabine in acetone.

It is to be understood that any reference to "3-(1,2,3,6-tetrahydropyridin-2-yl)pyridine glutarate" or "anatabine glutarate" herein is to be understood as also referring to any pharmaceutically acceptable solvate thereof.

The 3-(1,2,3,6-tetrahydropyridin-2-yl)pyridine glutarate has a chemical structure represented by the following formula (I):

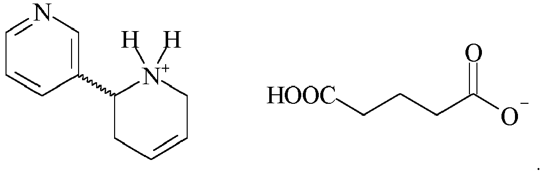

In a preferred embodiment, the 3-[1,2,3,6-tetrahydropyridin-2-yl]pyridine glutarate may thus have the following formula (Ia):

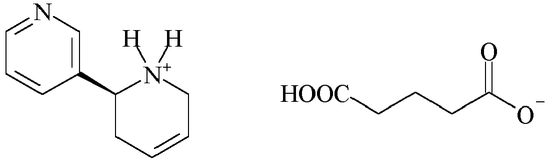

Preferably the anatabine glutarate is a specific polymorph (herein also referred to as polymorphic form) of the 3-(1,2,3,6-tetrahydropyridin-2-yl)pyridine glutarate and in particular of the crystal of the 3-(1,2,3,6-tetrahydropyridin-2-yl)pyridine glutarate. The polymorph preferably has an X-ray powder diffraction pattern (CuKα) substantially as shown in FIG. 1. The polymorph preferably has an X-ray powder diffraction pattern (CuKα) comprising one or more peaks selected from 8.0 ± 0.2 °2θ, 11.0 ± 0.2 °2θ, 13.3 ± 0.2 °2θ, 16.5 ± 0.2 °2θ, 18.0 ± 0.2 °2θ, 20.7 ± 0.2 °2θ, 21.0 ± 0.2 °2θ, 21.4 ± 0.2 °2θ, 22.0 ± 0.2 °2θ, 22.3 ± 0.2 °2θ, 23.3 ± 0.2 °2θ and 24.5 ± 0.2 °2θ. More preferably, the polymorph preferably has an X-ray powder diffraction pattern (CuKα) comprising one or more peaks selected from 8.0 ± 0.2 °2θ, 13.3 ± 0.2 °2θ, 16.5 ± 0.2 °2θ, 21.4 ± 0.2 °2θ, 22.0 ± 0.2 °2θ and 24.5 ± 0.2 °2θ.

Still more preferably, the polymorph preferably has an X-ray powder diffraction pattern (CuKα) comprising one or more peaks selected from 8.0 ± 0.1 °2θ, 11.0 ± 0.1 °2θ, 13.3 ± 0.1 °2θ, 16.5 ± 0.1 °2θ, 18.0 ± 0.1 °2θ, 20.7 ± 0.1 °2θ, 21.0 ± 0.1 °2θ, 21.4 ± 0.1 °2θ, 22.0 ± 0.1 °2θ, 22.3 ± 0.1 °2θ, 23.3 ± 0.1 °2θ and 24.5 ± 0.1 °2θ.Even more preferably, the polymorph preferably has an X-ray powder diffraction pattern (CuKα) comprising one or more peaks selected from 8.0 ± 0.1 °2θ, 13.3 ± 0.1 °2θ, 16.5 ± 0.1 °2θ, 21.4 ± 0.1 °2θ, 22.0 ± 0.1 °2θ and 24.5 ± 0.1 °2θ.

Even more specifically, the polymorph preferably has an X-ray powder diffraction pattern (CuKα) comprising one or more peaks selected from 7.960 ± 0.2 °2θ, 10.907 ± 0.2 °2θ, 13.291 ± 0.2 °2θ, 14.413 ± 0.2 °2θ, 15.239 ± 0.2 °2θ, 16.479 ± 0.2 °2θ, 17.933 ± 0.2 °2θ, 20.610 ± 0.2 °2θ, 20.977 ± 0.2 °2θ, 21.318 ± 0.2 °2θ, 21.927 ± 0.2 °2θ, 22.203 ± 0.2 °2θ, 22.792 ± 0.2 °2θ, 23.246 ± 0.2 °2θ, 24.426 ± 0.2 °2θ and 24.769 ± 0.2 °2θ.Still more specifically, the polymorph preferably has an X-ray powder diffraction pattern (CuKα) comprising one or more peaks selected from 7.960 ± 0.1 °2θ, 10.907 ± 0.1 °2θ, 13.291 ± 0.1 °2θ, 14.413 ± 0.1 °2θ, 15.239 ± 0.1 °2θ, 16.479 ± 0.1 °2θ, 17.933 ± 0.1 °2θ, 20.610 ± 0.1 °2θ, 20.977 ± 0.1 °2θ, 21.318 ± 0.1 °2θ, 21.927 ± 0.1 °2θ, 22.203 ± 0.1 °2θ, 22.792 ± 0.1 °2θ, 23.246 ± 0.1 °2θ, 24.426 ± 0.1 °2θ and 24.769 ± 0.1 °2θ.The above form of anatabine glutarate may be prepared using a method comprising the steps of:
a) preparing a solution comprising 3-[1,2,3,6-tetrahydropyridin-2-yl]pyridine, glutaric acid and a solvent,
b) allowing the formation of a salt of 3-[1,2,3,6-tetrahydropyridin-2-yl]pyridine with the glutaric acid, and
c) recovering the 3-[1,2,3,6-tetrahydropyridin-2-yl]pyridine glutaric acid salt.

The solvent used in the preparation of the solution of 3-[1,2,3,6-tetrahydropyridin-2-yl]pyridine, glutaric acid and a solvent preferably comprises 2-methyltetrahydrofuran, acetonitrile and/or ethyl acetate. More preferably, the solvent comprises 2-methyltetrahydrofuran.

The method may furthermore comprise a step of d) recrystallizing the 3-[1,2,3,6-tetrahydropyridin-2-yl]pyridine glutaric acid salt. Suitable solvents for this recrystallization include acetonitrile.

In step a), the anatabine glutarate can be prepared by combining anatabine free base, a solvent, and glutaric acid to create a reaction mixture. Anatabine glutarate typically forms in such a reaction mixture through contact of anatabine free base with glutaric acid. Preferably, anatabine free base as a 1 to 5 mass-% solution in acetonitrile is combined with glutaric acid.

Preferably a solution or suspension of anatabine free base, a solvent and glutaric acid is combined to form a reaction mixture, followed by precipitation and recovery of the anatabine glutarate salt from the mixture. Glutaric acid may be added either as a solid or as a solution or a suspension in a solvent.

The solvent is preferably selected from the group consisting of alkanols containing 1 to 8 carbon atoms, aliphatic esters containing 3 to 8 carbon atoms, aliphatic linear or cyclic ethers containing 3 to 8 carbon atoms, aliphatic ketones containing 3 to 8 carbon atoms, C₆₋₁₂ aromatic hydrocarbons (such as benzene and napthalene), acetonitrile, water, and any mixtures thereof. Preferably, the solvent is selected from aliphatic esters containing 3 to 8 carbon atoms, aliphatic cyclic ethers containing 3 to 8 carbon atoms, acetonitrile and a mixture thereof. More preferably, the solvent is selected from ethyl acetate, acetonitrile, 2-methyltetrahydrofuran, and any mixtures thereof. Even more preferably, the solvent contains acetonitrile. Still more preferably, the solvent is acetonitrile.

The anatabine free base, glutaric acid, and the at least one solvent are preferably combined to form the reaction mixture at about room temperature (i.e. a range of preferably 15°C to 25°C). The concentration of glutaric acid present in such reaction mixture is preferably a concentration close to the point of saturation (e.g. at least 80%, preferably 90%, more preferably 95% of the maximum achievable concentration). Anatabine glutarate typically precipitates out of the mixture. The precipitation may occur on its own or be induced, e.g., by the introduction of seed crystals. The reaction mixture may be stirred before, during, or after precipitation.

The reaction mixture may be heated and then cooled to facilitate precipitation of anatabine glutarate. Heating may be carried out up to any temperature (e.g. about 50°C to about 80°C) in the range of from room temperature to the boiling temperature of the solvent. Thereafter, cooling is generally conducted down to less than 40°C, preferably about 30°C to about 20°C, more preferably room temperature (i.e. a range of preferably 15°C to 25°C), to facilitate precipitation.

The resulting precipitate may be recovered by various techniques, such as filtration. The precipitate may be dried under ambient or reduced pressure and/or elevated temperature.

Anatabine glutarate, and particularly the polymorphic form described above, has advantageous properties such as high crystallinity, morphology, thermal and mechanical stability to polymorphic conversion and/or to dehydration, storage stability, low content of residual solvent, a lower degree of hygroscopicity, flowability, and advantageous processing and handling characteristics. Furthermore, anatabine glutarate recrystallizes as a crystalline salt even after having been exposed to moisture, when the moisture is removed by suitable measures, such as drying under vacuum.

Anatabine (for example, anatabine glutarate) can be administered to an individual to treat disorders comprising an inflammatory component, including chronic, low-level inflammation. Anatabine can be administered to an individual to reduce a symptom or a disorder comprising an NFKB-mediated inflammatory component and/or to reduce the risk of developing such a disorder. The NFKB-mediated inflammatory component may be associated with chronic inflammation which occurs, for example, in thyroiditis, cancer, arthritis, Alzheimer's disease, and multiple sclerosis. The inhalable powder comprising anatabine may have a monoamine oxidase (MAO) inhibitory effect. Additionally, or alternatively, the inhalable powder comprising anatabine may have a STAT3 phosphorylation inhibition effect.

In some embodiments, the anatabine is formulated as a salt. Any pharmaceutically acceptable salt may be used. Preferably, the anatabine salt is solid at room temperature (for example, solid at 25 °C). Suitable salts include, for example, a salt of aspartic acid ("aspartate"), gentisic acid ("gentisate"), benzoic acid ("benzoate"), fumaric acid ("fumarate"), hydrochloric acid ("hydrochlorate"), alfa-resorcylic acid ("alfa-resorcylate"), beta-resorcylic acid ("beta-resorcylate"), oxalic acid ("oxalate"), p-anisic acid ("anisate"), or glutaric acid ("glutarate"). Preferably the salt comprises glutarate, such as anatabine glutarate. Preferably, the anatabine salt is anatabine glutarate. Preferably, the anatabine glutarate is the polymorphic form described above.

Another preferred alkaloid is anabasine. Anabasine may preferably be an anabasine salt. Anabasine is a pyridine and piperidine alkaloid found in the tree tobacco plant.

Further pharmaceutically active ingredients include, for example: an antiviral compound such as acyclovir; anti-inflammatory compound such as salicylic acid, aceclofenac or ketoprofen; an antidiabetic compound such as metformin or glipizide; a antihypertensive compound such as oxprenolol; an antiemetic compound such as promethazine; an antidepressant compound such as seproxetine; an anticoagulant compound such as picotamide; a bronchodialator such as clenbuterol; or an anticancer compound such as beta-lapachone.

The beneficial crystallinity and reduced hygroscopicity may be achieved by selecting one or more parameters of the composition and the method of making the inhalable powder. For example, the alkaloid maybe selected such that it is solid at room temperature (for example, solid at 25°C). The alkaloid may be a solid at 30°C or below, or at 25°C.

The freeze drying and milling method forms composite particles each containing alkaloid and sugar alcohol. The alkaloid may be dispersed within a sugar alcohol matrix. Preferably the alkaloid is a solid stable salt at 25°C. Preferably the alkaloid is a solid stable salt at 25°C and is within a sugar alcohol crystalline matrix forming a particle or composite particle.

According to the present invention, the combining step preferably includes combining a sugar alcohol with an alkaloid and a liquid carrier to form a liquid mixture, and freeze drying the liquid mixture at a to form a crystalline dry composition. Then the crystalline dry composition is milled to form crystalline dry powder particles having a particle size of about 5 micrometres or less. At least selected crystalline dry powder particles may each comprise a solid alkaloid dispersed within a crystalline sugar alcohol matrix.

Sugars include lactose, which may be present in the solid powder as lactose monohydrate.

Sugar alcohols include, for example the following compounds and stereoisomers thereof, erythritol, adonitol, xylitol, arabitol, mannitol, sorbitol, myo-inositol, and the like. The sugar alcohol used in the present method includes erythritol, myo-inositol, adonitol, mannitol, and xylitol, or a combination thereof. Preferably the sugar alcohol is selected from erythritol (Tg=-44°C), mannitol (Tg=13°C), myo-inositol (Tg=50°C), stereoisomers thereof, and combinations thereof. Preferably the sugar alcohol is erythritol, mannitol, or myo-inositol.

The crystalline dry composition formed by freeze drying is milled to achieve a desired final particle size (for example, reduced from a particle size of about 50 micrometers to about 2 micrometers). The inhalable powder or crystalline dry powder particles may have a final particle size in a range from about 1 micrometre to about 5 micrometres, or from about 1 micrometre to about 3 micrometres, or from 1.5 micrometres to 2.5 micrometres.

The inhalable powder may further include an amino acid. Amino acid may be added to the alkaloid and sugar alcohol before the freeze drying step. Amino acid may be added to the crystalline dry powder particles after the freeze drying step. For example, the crystalline dry composition or particles formed by freeze drying may be co-milled (for example, jet-milled) with amino acid particles. The co-milling may cause the amino acid to at least partially coat the crystalline dry powder particles. The co-milling may further achieve a desired final particle size (for example, reduced from a particle size of about 50 micrometers to about 2 micrometers).

The combining step may preferably include combining a sugar alcohol with an alkaloid, an amino acid and a liquid carrier to form a liquid mixture, and freeze drying the liquid mixture to form a crystalline dry composition. Then the crystalline dry composition is milled to form crystalline dry powder particles having a particle size of about 5 micrometres or less. At least selected dry powder particles may each comprise an amino acid coating the crystalline dry powder particles comprising the solid alkaloid dispersed within a crystalline sugar matrix or crystalline sugar alcohol matrix.

The amino acid may comprise histidine, alanine, isoleucine, arginine, leucine, asparagine, lysine, aspartic acid, methionine, cysteine, phenylalanine, glutamic acid, threonine, glutamine, tryptophan, glycine, valine, pyrrolysine, proline, selenocysteine, serine, tyrosine, or a combination thereof. Preferably the amino acid comprises leucine, such as L-leucine.

The crystalline dry powder particles may comprise 5 wt-% or more or 10 wt-% or more of amino acid, and 30 wt-% or less or 25 wt-% or less of amino acid by weight of the crystalline dry powder particles. The crystalline dry powder particles may include from 5 wt-% to 30 wt-%, from 10 wt-% to 30 wt-%, from 15 wt-% to 25 wt-%, of amino acid by weight of the crystalline dry powder particles.

Preferably the crystalline dry powder particles may comprise 5 wt-% or more or 10 wt-% or more of leucine, and 30 wt-% or less or 25 wt-% or less of leucine by weight of the crystalline dry powder particles. The crystalline dry powder particles may include from 5 wt-% to 30 wt-%, from 10 wt-% to 30 wt-%, from 15 wt-% to 25 wt-%, of leucine by weight of the crystalline dry powder particles.

Providing an amino acid such as L-leucine with the crystalline dry powder particles may reduce adhesion forces of the crystalline dry powder particles and may reduce attraction between the particles and thus further reduce agglomeration of the particles.

The crystalline dry powder particles or crystalline dry powder particles may preferably form a homogenous population of particles, where each particle includes the alkaloid, sugar alcohol, and optional amino acid. The crystalline dry powder particles may each comprise from about 1 to about 10% alkaloid, from 99 to about 50% sugar alcohol, and optionally from 1 to about 40% amino acid. Preferably the crystalline dry powder particles may each comprise from about 1 to about 10% alkaloid, from 80 to about 70% sugar alcohol, and from 15 to about 25% amino acid.

The crystalline dry powder particles may each comprise from about 1 to about 10% alkaloid, from 99 to about 60% sugar alcohol, and optionally from 10 to about 30% amino acid. Preferably the crystalline dry powder particles may each comprise from about 1 to about 10% alkaloid, from 85 to about 65% sugar alcohol, and from 10 to about 30% amino acid.

The crystalline dry powder particles may each comprise from about 1 to about 10% nicotine, from 99 to about 60% sugar alcohol, and optionally from 10 to about 30% amino acid. Preferably the crystalline dry powder particles may each comprise from about 1 to about 10% nicotine, from 85 to about 65% sugar alcohol, and from 10 to about 30% amino acid.

The crystalline dry powder particles may comprise about 60% wt. or greater sugar alcohol, and 10% or greater amino acid and from about 1% to about 10% wt. solid alkaloid salt.

The crystalline dry powder particles may comprise a solid salt of an alkaloid; and a sugar alcohol comprising mannitol, erythritol, myo-inositol, adonitol, xylitol, or a combination thereof.

The combining step may comprise combining mannitol, erythritol or myo-inositol with an alkaloid compound and a liquid carrier to form a liquid mixture, and the freeze drying and milling steps form crystalline dry powder particles.

The combining step may comprise combining mannitol, erythritol or myo-inositol with a nicotine compound and a liquid carrier to form a liquid mixture, and the freeze drying and milling steps form crystalline dry powder particles.

The combining step may comprise combining mannitol, erythritol or myo-inositol with nicotine aspartate and a liquid carrier to form a liquid mixture, and the freeze drying and milling steps form crystalline dry powder particles.

The combining step may comprise combining mannitol, erythritol or myo-inositol with nicotine bitartrate and a liquid carrier to form a liquid mixture, and the freeze drying and milling steps form crystalline dry powder particles.

The combining step may comprise combining mannitol, erythritol or myo-inositol with nicotine glutarate and a liquid carrier to form a liquid mixture, and the freeze drying and milling steps form crystalline dry powder particles.

The combining step may comprise combining mannitol, erythritol or myo-inositol with nicotine malate and a liquid carrier to form a liquid mixture, and the freeze drying and milling steps form crystalline dry powder particles.

The combining step may comprise combining mannitol, erythritol or myo-inositol with anatabine glutarate and a liquid carrier to form a liquid mixture, and the freeze drying and milling steps form crystalline dry powder particles.

The combining step may comprise combining mannitol, erythritol or myo-inositol with anabasine and a liquid carrier to form a liquid mixture, and the freeze drying and milling steps form crystalline dry powder particles.

The method results in crystalline dry powder particles that contain the alkaloid and are suitable for inhalation. Advantageously, the method results in crystalline dry powder particles that exhibit low or reduced hygroscopicity. The method results in crystalline dry powder particles that do not readily absorb water or moisture when exposed to humid or tropical conditions. The method results in crystalline dry powder particles that do not readily agglomerate when exposed to humid or tropical conditions. Further advantageously, the method results in crystalline dry powder particles that are stable and possess a long shelf life.

The method of the present disclosure can be used to make inhalable powders with various alkaloids, as desired. In some embodiments, the alkaloid comprises an alkaloid. For example, the alkaloid may comprise nicotine or anatabine or anabasine.

The salt may be formed during the method (during the combining step) or may be formed before the combining step. For example, a free base alkaloid may be mixed with aspartic acid, gentisic acid, benzoic acid, fumaric acid, tartaric acid, lactic acid, maleic acid, muconic acid, hydrochloric acid, alfa-resorcylic acid, beta-resorcylic acid, oxalic acid, p-anisic acid, glutaric acid, or a combination thereof, preferably aspartic acid, tartaric acid or glutaric acid. The acid may be mixed with the free base alkaloid at any suitable ratio depending on the desired salt form and the rate of reaction. For example, a monoprotic acid such as benzoic acid may be combined with nicotine in about a 1:1 molar ratio to provide a monoprotonated nicotine salt.

The amount of alkaloid may be selected based on the desired or intended use of the inhalable powder. For example, the amount of alkaloid may be between 0.5 wt-% and 10 wt-% of the total weight of the crystalline dry powder particles. In some embodiments, the crystalline dry powder particles comprises 0.5 wt-% or more, 1 wt-% or more, 2 wt-% or more, or 3 wt-% or more of the alkaloid, and 12 wt-% or less, 10 wt-% or less, 9 wt-% or less, 8 wt-% or less, or 7 wt-% or less, of the alkaloid, or from 0.5 wt-% to 10 wt-%, from 1 wt-% to 8 wt-%, from 1.5 wt-% to 6 wt-%, or from 2 wt-% to 5 wt-of the alkaloid.

In some embodiments, the crystalline dry powder particles comprises 0.5 wt-% or more, 1 wt-% or more, 2 wt-% or more, or 3 wt-% or more of nicotine, and 12 wt-% or less, 10 wt-% or less, 9 wt-% or less, 8 wt-% or less, or 7 wt-% or less, of nicotine, or from 0.5 wt-% to 10 wt-%, from 1 wt-% to 8 wt-%, from 1.5 wt-% to 6 wt-%, or from 2 wt-% to 5 wt-% nicotine.

The amount of alkaloid may also be selected on a per-dose basis. The inhalable powder may be packaged in a single dose form or in a multiple dose form. For example, the inhalable powder may comprise 0.5 mg or more, 1 mg or more, 2 mg or more, or 5 mg or more of the alkaloid per dose. The inhalable powder may comprise 500 mg or less, 200 mg or less, 100 mg or less, 50 mg or less, 20 mg or less, or 10 mg or less of the alkaloid per dose. In some embodiments, the inhalable powder comprises from 0.01 to 10 mg of anatabine or nicotine or anabasine per dose, 0.05 to 5 mg anatabine or nicotine or anabasine per dose, or 0.1 to 1 mg of anatabine or nicotine or anabasine per dose.

The inhalable powder composition is made by a method comprising combining a sugar alcohol with the alkaloid and a liquid carrier to form a liquid mixture. The liquid mixture is freeze dried and then milled to form crystalline dry powder particles. Preferably the liquid carrier is aqueous. Preferably the liquid carrier is water. Preferably the liquid carrier is at least about 95 % water, or at least 99 % water, or 100 % water (based on total weight of liquid carrier).

The liquid carrier may also comprise an organic solvent, such as of an alkanol containing 1 to 8 carbon atoms, an aliphatic ester containing 3 to 8 carbon atoms, an aliphatic linear or cyclic ether containing 3 to 8 carbon atoms, an aliphatic ketone containing 3 to 8 carbon atoms, methanol, ethanol, acetone, or acetonitrile.

The liquid mixture may be freeze dried at a temperature and pressure to dehydrate the frozen liquid mixture via sublimation. Such freeze drying temperatures and pressure may be used for preparing a crystalline dry nicotine powder and to minimize the loss or degradation of nicotine during dehydration. The freeze drying temperature is the temperature of the atmosphere contacting the liquid mixture or dehydrating composition. The freeze drying pressure is the pressure of the atmosphere contacting the liquid mixture or dehydrating composition.

The freeze drying step may include two or more changes in temperature or pressure for prescribed time intervals. The freeze drying step may include: an initial freeze interval, then a primary drying interval, and then a secondary drying interval.

An initial freeze interval may cool the liquid mixture below its triple point to ensure sublimation rather than melting occurs during the subsequent freeze drying intervals or steps. The initial freeze interval may have a pressure that is about standard pressure or atmospheric pressure.

The initial freeze interval may reduce the temperature of the liquid mixture to a temperature at least 10 degrees Celsius below its triple point, or at least 15 degrees Celsius below its triple point, or at least 20 degrees Celsius below its triple point, or in a range from 10 to 30 degrees Celsius below its triple point, or in a range from 15 to 25 degrees Celsius below its triple point, or about 20 Celsius below its triple point. The triple point for water is about 0 degrees Celsius.

The initial freeze interval may be from 5 hours to 40 hours to ensure complete cooling of the liquid mixture below its triple point. The initial freeze interval may be from 10 hours to 30 hours. The initial freeze interval may be from 15 hours to 25 hours. The initial freeze interval may be about 20 hours.

The initial freeze interval may be in a range from 10 to 30 degrees Celsius below its triple point for a time interval from 10 hours to 30 hours. The initial freeze interval may be in a range from 15 to 25 degrees Celsius below its triple point for a time interval from 15 hours to 25 hours. The initial freeze interval may be about 20 degrees Celsius below its triple point for a time interval of about 20 hours.

A primary drying interval may cool the liquid mixture further below its triple point and reduce the pressure to a substantial vacuum to ensure sublimation rather than melting occurs. A bulk of the ice is removed with the primary drying interval. Pressure during the primary drying interval may range from 0.5 mbar to 100 mbar, or from 0.5 mbar to 50 mbar, or 0.5 mbar to 10 mbar, or from 0.5 mbar to 1 mbar, or 0.5 mbar to 0.7 mbar.

The primary drying interval may reduce the temperature of the liquid mixture to a temperature of at least 20 degrees Celsius below its triple point, or at least 30 degrees Celsius below its triple point, or at least 40 degrees Celsius below its triple point, or in a range from 20 to 60 degrees Celsius below its triple point, or in a range from 30 to 50 degrees Celsius below its triple point, or about 40 degrees Celsius below its triple point.

The primary drying interval may be from 1 hour to 15 hours. The primary drying interval may be from 2 hours to 10 hours. The primary drying interval may be from 4 hours to 8 hours. The primary drying interval may be about 6 hours.

The primary drying interval may be in a range from 30 to 50 degrees Celsius below its triple point for a time interval from 2 hours to 10 hours. The primary drying interval may be about 40 degrees Celsius below its triple point for a time interval of about 6 hours.

The secondary drying interval removes remaining frozen liquid carrier from the composition resulting in the crystalline dry composition. The secondary drying interval may ramp up the primary drying temperature slowly over the time interval. Pressure during the secondary drying interval may range from 0.5 mbar to 100 mbar, or from 0.5 mbar to 50 mbar, or 0.5 mbar to 10 mbar, or from 0.5 mbar to 1 mbar, or 0.5 mbar to 0.7 mbar.

Secondary drying temperature may ramp up at a rate of 1 or 2 or 3 or 4 degrees Celsius per hour of the secondary drying interval. The secondary drying temperature may start at the primary drying temperature and end at the triple point to 40 degrees Celsius above the triple point, or from 10 to 30 degrees Celsius above its triple point, or from 15 to 25 degrees Celsius above its triple point, or about 20 degrees Celsius above its triple point. Alternatively, the secondary drying temperature may start at the primary drying temperature and end at about 25 degrees Celsius.

The secondary drying interval may be from 15 hour to 50 hours. The secondary drying interval may be from 20 hours to 40 hours. The secondary drying interval may be from 25 hours to 35 hours. The secondary drying interval may be about 30 hours.

When the liquid carrier is water, the secondary drying interval may be in a ramp up from - 30 degrees Celsius to -50 degrees Celsius to a temperature from 10 degrees Celsius to 30 degrees Celsius for a time interval from 20 hours to 40 hours. The secondary drying interval may be in a ramp up from -30 degrees Celsius to -50 degrees Celsius to a temperature from 15 degrees Celsius to 25 degrees Celsius for a time interval from 25 hours to 35 hours.

The crystalline dry composition may then be held for a hold interval at a temperature from 10 degrees Celsius to 30 degrees Celsius for a time interval from 20 hours to 40 hours. The hold interval may have a pressure that is about standard pressure or atmospheric pressure. Alternately, the hold interval may have a pressure that is range from 0.5 mbar to 100 mbar, or from 0.5 mbar to 50 mbar, or 0.5 mbar to 10 mbar, or from 0.5 mbar to 1 mbar, or 0.5 mbar to 0.7 mbar.

The crystalline dry composition may then be milled to form the crystalline dry powder particles described herein.

As a result of being freeze dried from a liquid mixture of the alkaloid and sugar alcohol and then milled, the crystalline dry powder particles contain alkaloid that is dispersed throughout the particle in a sugar alcohol matrix. The matrix of crystalline sugar alcohol may help protect the alkaloid from environmental factors, such as high temperature and humidity. The matrix of crystalline sugar alcohol also helps minimize agglomeration and maintain good flowability and aerodynamic properties of the crystalline dry powder particles. However, once the crystalline dry powder particles reach the lungs of a user, the crystalline dry powder particles dissolve rapidly, which helps to provide fast uptake of the alkaloid.

The crystalline dry powder particles have a particle size of 5 µm or less, or ranging from 0.5 µm to 5 µm, or from 0.75 µm to 5 µm, or from 1 µm to 5 µm, or from 1 µm to 3 µm, or from 1.5 µm to 2.5 µm. The desired particle size range may be achieved by freeze drying, milling, sieving, or a combination thereof.

The crystalline dry powder particles may have pH (in solution) in a range recommended for human consumption. In some embodiments, the crystalline dry powder particles have a pH of 6 or less, 7 or less, or 8 or less, or between 3 and 8, or between 3 and 6, or between 6 and 8 when dissolved in water. The pH of the crystalline dry powder particles may be measured by reconstituting the powder in deionized water at a concentration of 1 mg/ml and measuring the pH of the resulting solution at standard temperature and pressure. The crystalline dry powder particles may be formulated without the use of an additional buffer. Additional buffering agents may be considered to be compounds capable of buffering (for example, salts, acids, bases, and combinations thereof) other than the acid used to form the salt with the alkaloid, or the amino acid included in the crystalline dry powder particles. The crystalline dry powder particles may be free of surfactants.

The crystalline dry powder particles may be further mixed with a second population of particles to form a powder system. Preferably, the second population of particles have a different particle size or larger particle size than the crystalline dry powder particles. For example, the second population of particles may have a particle size of about 20 µm or greater, or about 50 µm or greater, 200 µm or smaller, 150 µm or smaller, or in a range from 50 µm to 200 µm, or from 50 µm to 150 µm. The second population of particles may have any useful size distribution for inhalation delivery selectively into the mouth or buccal cavity of a user. The larger second population of flavourant particles may assist in delivery of the crystalline dry powder particles to the inhalation airflow to the user.

The crystalline dry powder particles and second population of particles may be combined in any useful relative amount so that the second population of particles are detected by the user when consumed with the crystalline dry powder particles. Preferably, the crystalline dry powder particles and second population of particles form at least about 90 wt-% or at least about 95 wt-% or at least about 99 wt-% or 100 wt-% of the total weight of the powder system.

The crystalline dry powder particles may be further mixed with a second population of flavourant particles to form a powder system. Preferably, the second population of flavourant particles have a different particle size or larger particle size than the crystalline dry powder particles. For example, the flavor particles may have a particle size of about 20 µm or greater, or about 50 µm or greater, 200 µm or smaller, 150 µm or smaller, or in a range from 50 µm to 200 µm, or from 50 µm to 150 µm. The second population of flavourant particles may have any useful size distribution for inhalation delivery selectively into the mouth or buccal cavity of a user. The larger second population of flavourant particles may assist in delivery of the crystalline dry powder particles to the inhalation airflow to the user.

The crystalline dry powder particles and second population of flavourant particles may be combined in any useful relative amount so that the second population of flavourant particles are detected by the user when consumed with the crystalline dry powder particles. Preferably, the crystalline dry powder particles and second population of flavourant particles form at least about 90 wt-% or at least about 95 wt-% or at least about 99 wt-% or 100 wt-% of the total weight of the powder system.

The crystalline dry powder particles or powder system may be provided in a suitable dosage form. For example, the crystalline dry powder particles or powder system may be provided in a capsule. The dosage form (for example, capsule) may be configured for use in a suitable inhaler. For example, the capsule may be utilized in an inhaler device having a capsule cavity. Air flow management through a capsule cavity of the inhaler device may cause a capsule contained therein to rotate during inhalation and consumption. The capsule may contain crystalline dry powder particles or powder system

According to an embodiment, the crystalline dry powder particles or powder system is formulated to have reduced hygroscopicity and reduced tendency to agglomerate or clump together if water or moisture contacts the crystalline dry powder particles. Rotation of a pierced capsule may suspend and aerosolize the crystalline dry powder particles or powder system released from the pierced capsule into the inhalation air moving through the inhaler device. The optional flavor particles may be larger than the crystalline dry powder particles and may assist in transporting the crystalline dry powder particles to the user while the flavor particles preferentially deposit in the mouth or buccal cavity of the user. The crystalline dry powder particles and optional flavor particles may be delivered with the inhaler device at inhalation or air flow rates that are within conventional smoking regime inhalation or air flow rates.

The crystalline dry powder particles may comprise a therapeutically effective dose of the alkaloid, such as anatabine glutarate. Anatabine (for example, anatabine glutarate) can be administered to an individual to reduce a symptom or a disorder comprising an NFKB-mediated inflammatory component and/or to reduce the risk of developing such a disorder. The NFKB-mediated inflammatory component may be associated with chronic inflammation which occurs, for example, in thyroiditis, cancer, arthritis, Alzheimer's disease, and multiple sclerosis. The crystalline dry powder particles comprising anatabine may have a monoamine oxidase (MAO) inhibitory effect. Additionally or alternatively, the crystalline dry powder particles comprising anatabine may have a STAT3 phosphorylation inhibition effect.

The sugar alcohol is selected from erythritol, myo-inositol, adonitol, mannitol, and xylitol, or a combination thereof.

Preferably the sugar alcohol is selected from erythritol, myo-inositol, and mannitol. Preferably the sugar alcohol is erythritol or myo-inositol.

The sugar alcohol may have a solubility of 75 g or less, 60 g or less, 50 g or less, or 30 g or less in 100 g of water at 25°C.

The crystalline dry powder particles may exhibit a hygroscopicity of about 5% or less. The crystalline dry powder particles may exhibit a hygroscopicity of about 4% or less. The crystalline dry powder particles may exhibit a hygroscopicity of about 3% or less. The crystalline dry powder particles may exhibit a hygroscopicity of about 2.5% or less. The crystalline dry powder particles may exhibit a hygroscopicity of about 2% or less. The crystalline dry powder particles may exhibit a hygroscopicity of about 1.5% or less. The crystalline dry powder particles may exhibit a hygroscopicity of about 1% or less. The crystalline dry powder particles may exhibit a hygroscopicity in a range from 0% to about 5%, or 0% to about 4%, or 0% to about 3%, or 0% to about 2%.

Hygroscopicity may be measured by measuring the water uptake or absorption (wt% increase) of the sample at tropical conditions (75% relative humidity and 30 degrees Celsius atmosphere) over a 20-minute time duration. Prior to exposure to tropical condition, the sample is equilibrated at 0% relative humidity, and 25 degrees Celsius atmosphere for 24 hours to reduce or remove non-bonded water from the sample. The hygroscopicity value is measured utilizing a "Vapor Sorption Analyzer SPSx-1u High Load" manufactured by ProUmid GmbH & Co. KG, as described in the Examples below.

The crystalline dry powder particles may be made by mixing the alkaloid with an acid or base, the sugar alcohol, and the liquid carrier into a flowable liquid mixture, and freeze drying the flowable mixture. The use of an acid or base may lead to the formation of a salt of the alkaloid which is solid at room temperature. This may be advantageous as it allows the incorporation of alkaloids which are not solid at room temperature or may provide a solid with preferable physical properties. The method may further comprise adding an amino acid to the crystalline dry powder particles or flowable liquid mixture. The method may further comprise combining an amino acid with the sugar, alkaloid, and acid to form the mixture. The method may further comprise adding an amino acid to both the liquid mixture and to the particles.

The crystalline dry powder particles may be made by mixing the alkaloid with an acid, the sugar alcohol, and the liquid carrier into a flowable liquid mixture, and freeze drying the flowable mixture. The use of an acid may lead to the formation of a salt of the alkaloid which is solid at room temperature. This may be advantageous as it allows the incorporation of alkaloids which are not solid at room temperature or may provide a solid with preferable physical properties. This has been found to be particularly useful when the alkaloid is nicotine or anatabine or anabasine. The method may further comprise adding an amino acid to the crystalline dry powder particles. The method may further comprise combining an amino acid with the sugar, alkaloid, and acid to form the mixture. The method may further comprise adding an amino acid to both the liquid mixture and to the crystalline dry powder particles.

Preferably the method comprises combining mannitol, erythritol, or myo-inositol with an alkaloid and water to form a liquid mixture, and freeze drying the liquid mixture and then milling the crystalline dry composition to form crystalline dry powder particles.

Preferably the method comprises combining mannitol, erythritol, or myo-inositol with nicotine and water to form a liquid mixture, and freeze drying the liquid mixture and then milling the crystalline dry composition to form crystalline dry powder particles.

Preferably the method comprises combining mannitol, erythritol, or myo-inositol with anatabine and water to form a liquid mixture, and freeze drying the liquid mixture and then milling the crystalline dry composition to form crystalline dry powder particles.

Preferably the method comprises combining mannitol, erythritol, or myo-inositol with anabasine and water to form a liquid mixture, and freeze drying the liquid mixture and then milling the crystalline dry composition to form crystalline dry powder particles.

Preferably at least selected crystalline dry powder particles each comprise a solid alkaloid salt dispersed within a crystalline mannitol matrix, crystalline erythritol matrix, or crystalline myo-inositol matrix.

Preferably at least selected crystalline dry powder particles each comprise a solid nicotine salt dispersed within a crystalline mannitol matrix, crystalline erythritol matrix, or crystalline myo-inositol matrix.

The crystalline dry powder particles may comprise nicotine aspartate; mannitol, myo-inositol, or erythritol; and leucine. The crystalline dry powder particles may comprise from 1 wt-% to 8 wt-% nicotine aspartate, from 70 wt-% to 99 wt-% mannitol, myo-inositol, or erythritol, and from 0 wt-% to 29 wt-% leucine. The crystalline dry powder particles may comprise from 1 wt-% to 8 wt-% nicotine aspartate, from 70 wt-% to 80 wt-% mannitol, myo-inositol, or erythritol, and from 15 wt-% to 25 wt-% leucine.

The crystalline dry powder particles may comprise nicotine bitartrate; mannitol, myo-inositol, or erythritol; and leucine. The crystalline dry powder particles may comprise from 1 wt-% to 8 wt-% nicotine bitartrate, from 70 wt-% to 80 wt-% mannitol, myo-inositol, or erythritol, and from15 wt-% to 25 wt-% leucine.

The crystalline dry powder particles may comprise nicotine glutarate; mannitol, myo-inositol, or erythritol; and leucine. The crystalline dry powder particles may comprise from 1 wt-% to 8 wt-% nicotine glutarate, from 70 wt-% to 80 wt-% mannitol, myo-inositol, or erythritol, and from 15 wt-% to 25 wt-% leucine.

The crystalline dry powder particles may comprise nicotine malate; mannitol, myo-inositol, or erythritol; and leucine. The crystalline dry powder particles may comprise from 1 wt-% to 8 wt-% nicotine malate, from 70 wt-% to 80 wt-% mannitol, myo-inositol, or erythritol, and from 15 wt-% to 25 wt-% leucine.

The crystalline dry powder particles may comprise anatabine glutarate; mannitol, myo-inositol, or erythritol; and leucine. The crystalline dry powder particles may comprise from 1 wt-% to 10 wt-% anatabine glutarate, from 70 wt-% to 80 wt-% mannitol, myo-inositol, or erythritol, and from 15 wt-% to 25 wt-% leucine.

In some embodiments, the crystalline dry powder particles may be free of mannitol when the alkaloid is aspartate.

The method of the present disclosure results in a matrix of crystalline sugar that helps protect the alkaloid from environmental factors, such as high temperature and humidity. The matrix of crystalline sugar also helps minimize agglomeration and maintain good flowability and aerodynamic properties of the inhalable powder. However, once the inhalable powder reaches the lungs of a user, the particles dissolve rapidly, which helps to provide fast uptake of the alkaloid.

The method also enables high throughput manufacturing of the inhalable powder without compromising the physical and chemical properties of the crystalline dry powder particles or of the alkaloid.

The lower hygroscopicity of the crystalline dry powder particles may help minimize losses of the composition due to agglomeration, stickiness, or liquification. This may provide improved control of the particle size of the crystalline dry powder particles, the size of the dose and the chemical composition of the crystalline dry powder particles delivered to the consumer. In addition to having lower hygroscopicity and thus increased shelf life, the crystalline dry powder particles may advantageously have improved aerodynamic properties. For example, a consumer may be able to consume more than half the crystalline dry powder particles contained in a cartridge or capsule. Because the crystalline dry powder particles does not readily absorb water, a consumer may be able to get more usage sessions or puffs from a single cartridge or capsule. The crystalline dry powder particles may also be provided in larger dosage forms, such as cartridges or capsules, due to the fact that the quality of the crystalline dry powder particles is not compromised soon after opening of the package.

The term "particle size" is used here to refer to the mass median aerodynamic diameter (MMAD) of the particle or set of particles, unless otherwise stated. Such values are based on the distribution of the aerodynamic particle diameters defined as the diameter of a sphere with a density of 1 gm/cm³ that has the same aerodynamic behavior as the particle which is being characterized.

In particular, for a powder system reference is commonly made to the mass median aerodynamic diameter (MMAD), one of the metrics most widely adopted as a single number descriptor of aerodynamic particle-size distribution. The MMAD is a statistically derived figure for a particle sample: by way of example, an MMAD of 5 micrometres means that 50 percent of the total sample mass will be present in particles having aerodynamic diameters of less than 5 micrometres, and that the remaining 50 percent of the total sample mass will be present in particles having an aerodynamic diameter greater than 5 micrometres. In the context of the present invention, when describing a powder system, the term "particle size" refers to the MMAD of the powder system.

The MMAD of a powder system is measured with a cascade impactor. Cascade impactors are instruments which have been extensively used for sampling and separating airborne particles for determining the aerodynamic size classification of aerosol particles. In practice, cascade impactors separate an incoming sample into discrete fractions on the basis of particle inertia, which is a function of particle size, density and velocity. A cascade impactor typically comprises a series of stages, each of which comprises a plate with a specific nozzle arrangement and a collection surface. As nozzle size and total nozzle area both decrease with increasing stage number, the velocity of the sample-laden air increases as it proceeds through the instrument. At each stage, particles with sufficient inertia break free from the prevailing air stream to impact on the collection surface. Therefore, at any given flow rate, each stage is associated with a cut-off diameter, a figure that defines the size of particles collected. With increasing stage number, velocity increases and so stage cut-off diameter decreases. Thus, the cut-off diameter associated with a given stage is a function of the air-flow rate used for testing. To reflect in-use performance, nebulisers are routinely tested at 15 L/min and dry powder inhalers may be tested at flow rates up to 100 L/min.

Preferably, in the context of the present invention, the MMAD of a powder system is measured with a Next Generation Impactor (NGI) 170 (available from Copley Scientific AG). The NGI is a high performance, precision, particle classifying cascade impactor having seven stages plus a Micro-Orifice Collector (MOC). Characteristics and operation principle of a NGI are described, for example, in Marple et al., Journal of Aerosol Medicine - Volume 16, Number 3 (2003). More preferably, measurements are carried out at 20 ±3 degrees Celsius and relative humidity of 35 ± 5 percent.

A dry powder formulation typically contains less than or equal to about 15 percent by weight moisture, preferably less than or equal to about 10 percent moisture, even more preferably less than or equal to about 6 percent by weight moisture. Most preferably a dry powder formulation contains less than or equal to about 5 percent by weight moisture or even less than or equal to about 3 percent by weight moisture or even less than or equal to about 1 percent by weight moisture.

All values reported as a percentage are presumed to be weight percent based on the total weight.

All scientific and technical terms used herein have meanings commonly used in the art unless otherwise specified. The definitions provided herein are to facilitate understanding of certain terms used frequently herein.

As used herein, the singular forms "a", "an", and "the" encompass embodiments having plural referents, unless the content clearly dictates otherwise.

As used herein, "or" is generally employed in its sense including "and/or" unless the content clearly dictates otherwise. The term "and/or" means one or all of the listed elements or a combination of any two or more of the listed elements.

As used herein, "have", "having", "include", "including", "comprise", "comprising" or the like are used in their open-ended sense, and generally mean "including, but not limited to". It will be understood that "consisting essentially of", "consisting of", and the like are subsumed in "comprising," and the like.

The words "preferred" and "preferably" refer to embodiments of the invention that may afford certain benefits, under certain circumstances. However, other embodiments may also be preferred, under the same or other circumstances. Furthermore, the recitation of one or more preferred embodiments does not imply that other embodiments are not useful and is not intended to exclude other embodiments from the scope of the disclosure, including the claims.

The term "substantially" as used here has the same meaning as "significantly," and can be understood to modify the relevant term by at least about 90 %, at least about 95 %, or at least about 98 %. The term "not substantially" as used here has the same meaning as "not significantly," and can be understood to have the inverse meaning of "substantially," i.e., modifying the relevant term by not more than 10 %, not more than 5 %, or not more than 2 %.

The invention is defined in the claims.

The Examples will now be further described with reference to the figures in which:
FIG. 1 is an X-ray powder diffraction pattern (CuKα) of a preferred polymorph of anatabine glutarate.
FIG. 2 is a schematic diagram of an illustrative inhaler article.

The illustrative inhaler article 10 of FIG. 2 includes an elongated cylindrical body extending from a mouthpiece end 11 to a distal end 12. A capsule cavity 20 is defined within the elongated cylindrical body. A capsule 30 is contained within the capsule cavity 20. Crystalline dry powder particles described herein may be contained within the capsule 30. The capsule 30 may be pierced to form an aperture through the body of the capsule 30 and inhalation air may flow through the inhaler article 10 to release crystalline dry powder particles from the pierced capsule 30 and into the inhalation airflow and out of the mouthpiece end 11.

The schematic drawings are not necessarily to scale and are presented for purposes of illustration and not limitation. The drawings depict one or more aspects described in this disclosure. However, it will be understood that other aspects not depicted in the drawing fall within the scope of this disclosure.

### EXAMPLES

A first liquid mixture was formed by combining 2.5% free base nicotine, 20% leucine, 73.4% erythritol, and 4.1% aspartic acid in water.

A second liquid mixture was formed by combining 7.7% nicotine bitartrate (2.5% nicotine content), 20% leucine, and 73.4% erythritol in water.

A third liquid mixture was formed by combining 2.5% free base nicotine, 20% leucine, 73.4% erythritol, and 2.1% glutaric acid in water.

The first and second liquid mixtures were then each freeze dried according to the Table 1 below.

**Table 1**

| Parameter | Equipment | Setting | Duration |
|---|---|---|---|
| Initial Freeze | Lab Freezer | -20°C | ∼21 hours |
| Primary Drying | Edwards Supermodulyo Freeze Dryer | -40°C | ∼6 hours |
| Secondary Drying | | -40°C up to 20°C +2°C per hour | ∼30 hours |
| Hold | | 20°C | ∼36 hours |

A vacuum pressure of about 0.625--0.650 mbar was applied to the drying intervals. The freeze drying chamber was pressurized by purging with nitrogen gas and forming the crystalline dry composition. The crystalline dry composition was then milled using an Atritor M3 fluid energy mill. The resulting crystalline dry particles produced a fine, free-flowing white powder.

The crystalline dry particles (Sample A) produced from the first liquid mixture had a particle size distribution of: X₁₀=0.71 micrometer, X₅₀=1.73 micrometers, X₉₀=3.60 micrometers and a VMD=1.98.

The crystalline dry particles (Sample B) produced from the second liquid mixture had a particle size distribution of: X₁₀=0.72 micrometer, X₅₀=1.75 micrometers, X₉₀=3.68 micrometers and a VMD=2.02.

A first comparative sample "Comp Ex1" is produced utilizing an amorphous sugar trehalose (solubility 69g/100 ml water at 25 °C) utilizing a comparative liquid mixture formed by combining 2.5% free base nicotine, 10% leucine, 86.25% trehalose, 1.25% lactic acid in water. The comparative liquid mixture was fed to a spray dryer (Buchi B-290) operating with a first condition set (5.5 bar, 70°C and 3 g/min feed rate) to form amorphous dry powder particles.

A second comparative sample "Comp Ex2" is produced utilizing the first liquid mixture above but spray dried and milled to form the crystalline dry powder particles. The first liquid mixture was fed to a spray dryer (Buchi B-290) operating with a first condition set (3.0 bar, 50°C and 2.5 g/min feed rate) to form crystalline dry powder particles.

The crystalline dry particles (Sample A) produced from the first liquid mixture, and the first comparative sample "Comp Ex1", and the second comparative sample "Comp Ex2" are tested using an aerosol generation test under tropical conditions.

About 50 grams of each sample powder was loaded into a capsule and the capsule was placed into an inhaler device and pierced. Inhalation air (75% relative humidity and 30 degrees Celsius) was passed through the inhaler at a puff rate of 80 ml per every two seconds for a total of 20 puffs. The inhaler device was weighed before and after the aerosol generation test to determine the amount of each sample that was released from the capsule.
- Sample A released 29 grams.
- The comparative sample "Comp Ex2" released 26 grams.
- The comparative sample "Comp Ex1" actually increased in weight by 0.1 grams, indicating that more water was absorbed than sample released.

Additional crystalline dry powder particles are formed (utilizing the formulations listed in Table 2 below) utilizing the freeze drying and milling parameters set forth in this example above.

**Table 2**

| Sample | Nicotine % | Aspartic Acid % | Leucine % | Sugar or Sugar Alcohol % |
|---|---|---|---|---|
| A | 2.5 | 4.1 | 20 | 73.4 - Mannitol |
| B | 2.5 | 4.1 | 20 | 73.4 - Myo-Inositol |
| C | 2.5 | 4.1 | 20 | 73.4 - Adonitol |
| D | 2.5 | 4.1 | 20 | 73.4 - Lactose Monohydrate |
| E | 2.5 | 4.1 | 20 | 73.4 - Xylitol |

The method to prepare sample D is outside the scope of the invention. Powder samples A-E all exhibit crystalline structure.

For the purpose of the present description and of the appended claims, except where otherwise indicated, all numbers expressing amounts, quantities, percentages, and so forth, are to be understood as being modified in all instances by the term "about."

Within this context, a number A may be considered to include numerical values that are within general standard error for the measurement of the property that the number A modifies.

## Claims

1. A method of forming an inhalable powder composition comprising:
combining a sugar alcohol with an alkaloid and a liquid carrier to form a liquid mixture, the sugar alcohol comprising erythritol, myo-inositol, adonitol, mannitol, and xylitol, or a combination thereof;
freeze drying the liquid mixture to form a crystalline dry composition; and
milling the crystalline dry composition to form crystalline dry powder particles having a mass median aerodynamic diameter (MMAD) of about 5 micrometers or less when measured with a cascade impactor.

2. The method of claim 1, wherein the combining step comprises combining the sugar alcohol with an alkaloid and an amino acid and a liquid carrier to form a liquid mixture.

3. The method of any preceding claim, wherein the milling step comprises jet milling the crystalline dry composition to form crystalline dry powder particles.

4. The method of any preceding claim, wherein the milling step comprises milling the crystalline dry composition to form crystalline dry powder particles having a mass median aerodynamic diameter (MMAD) in a range from about 1 micrometer to about 3 micrometers, or from 1.5 micrometers to 2.5 micrometers when measured with a cascade impactor.

5. The method of any preceding claim, wherein the alkaloid comprises a salt of an alkaloid.

6. The method of any preceding claim, wherein the alkaloid comprises nicotine bitartrate, or nicotine aspartate, nicotine malate, or nicotine glutarate.

7. The method of any preceding claim, wherein the alkaloid comprises an anatabine salt.

8. The method of any preceding claim, wherein the crystalline dry powder particles comprise about 60% wt. or greater sugar alcohol, and 10% wt. or greater amino acid and from about 1% to about 10% wt. solid alkaloid salt.

9. The method of any preceding claim wherein the combining step comprises combining mannitol, erythritol or myo-inositol with an alkaloid compound and water to form a liquid mixture.

10. The method of any preceding claim wherein the combining step comprises combining mannitol, erythritol or myo-inositol with a nicotine compound and water to form a liquid mixture.

11. The method of any preceding claim wherein the combining step comprises combining mannitol, erythritol or myo-inositol with an anatabine compound and water to form a liquid mixture.

12. The method of any preceding claim wherein the crystalline dry powder particles exhibit a hygroscopicity of about 5% or less, or 4% or less, or 3% or less, or 2% or less, or 1% or less.

13. The method of any preceding claim wherein an amino acid is added to the crystalline dry powder particles after the freeze drying step.

14. The method of claim 13 wherein the milling step comprises co-milling the crystalline dry powder particles with amino acid particles.

15. The method of claim 2, 8, 13 or 14, wherein the amino acid comprises leucine.

## Patentansprüche

1. Verfahren zum Bilden einer inhalierbaren Pulverzusammensetzung, umfassend:
Kombinieren eines Zuckeralkohols mit einem Alkaloid und einem flüssigen Träger, um ein flüssiges Gemisch zu bilden, wobei der Zuckeralkohol Erythrit, Myo-Inosit, Adonit, Mannit und Xylit oder eine Kombination davon umfasst;
Gefriertrocknen des flüssigen Gemischs, um eine kristalline Trockenzusammensetzung zu bilden; und
Mahlen der kristallinen Trockenzusammensetzung, um kristalline Trockenpulverpartikel mit einem mittleren aerodynamischen Massendurchmesser (MMAD) von etwa 5 Mikrometer oder weniger, bei Messung mit einem Kaskadenimpaktor, zu bilden.

2. Verfahren nach Anspruch 1, wobei der Kombinierschritt ein Kombinieren des Zuckeralkohols mit einem Alkaloid und einer Aminosäure und einem flüssigen Träger, um ein flüssiges Gemisch zu bilden, umfasst.

3. Verfahren nach einem beliebigen vorhergehenden Anspruch, wobei der Mahlschritt ein Strahlmahlen der kristallinen Trockenzusammensetzung, um kristalline Trockenpulverpartikel zu bilden, umfasst.

4. Verfahren nach einem beliebigen vorhergehenden Anspruch, wobei der Mahlschritt ein Mahlen der kristallinen Trockenzusammensetzung, um kristalline Trockenpulverpartikel mit einem mittleren aerodynamischen Massendurchmesser (MMAD) in einem Bereich von etwa 1 Mikrometer bis etwa 3 Mikrometer oder von 1,5 Mikrometer bis 2,5 Mikrometer, bei Messung mit einem Kaskadenimpaktor, zu bilden.

5. Verfahren nach einem beliebigen vorhergehenden Anspruch, wobei das Alkaloid ein Salz eines Alkaloids umfasst.

6. Verfahren nach einem beliebigen vorhergehenden Anspruch, wobei das Alkaloid Nikotinbitartrat oder Nikotinaspartat, Nikotinmalat oder Nikotinglutarat umfasst.

7. Verfahren nach einem beliebigen vorhergehenden Anspruch, wobei das Alkaloid ein Anatabinsalz umfasst.

8. Verfahren nach einem beliebigen vorhergehenden Anspruch, wobei die kristallinen Trockenpulverpartikel etwa 60 Gew.-% oder mehr Zuckeralkohol und 10 Gew.-% oder mehr Aminosäure und etwa 1 Gew.-% bis etwa 10 Gew.-% festes Alkaloidsalz umfassen.

9. Verfahren nach einem beliebigen vorhergehenden Anspruch, wobei der Kombinierschritt ein Kombinieren von Mannit, Erythrit oder Myo-Inosit mit einer Alkaloidverbindung und Wasser, um ein flüssiges Gemisch zu bilden, umfasst.

10. Verfahren nach einem beliebigen vorhergehenden Anspruch, wobei der Kombinierschritt ein Kombinieren von Mannit, Erythrit oder Myo-Inosit mit einer Nikotinverbindung und Wasser, um ein flüssiges Gemisch zu bilden, umfasst.

11. Verfahren nach einem beliebigen vorhergehenden Anspruch, wobei der Kombinierschritt ein Kombinieren von Mannit, Erythrit oder Myo-Inosit mit einer Anatabinverbindung und Wasser, um ein flüssiges Gemisch zu bilden, umfasst.

12. Verfahren nach einem beliebigen vorhergehenden Anspruch, wobei die kristallinen Trockenpulverpartikel eine Hygroskopizität von etwa 5 % oder weniger oder 4 % oder weniger oder 3 % oder weniger oder 2 % oder weniger oder 1 % oder weniger aufweisen.

13. Verfahren nach einem beliebigen vorhergehenden Anspruch, wobei den kristallinen Trockenpulverpartikeln nach dem Gefriertrocknungsschritt eine Aminosäure hinzugefügt wird.

14. Verfahren nach Anspruch 13, wobei der Mahlschritt ein gemeinsames Mahlen der kristallinen Trockenpulverpartikel mit Aminosäurepartikeln umfasst.

15. Verfahren nach Anspruch 2, 8, 13 oder 14, wobei die Aminosäure Leucin umfasst.

## Revendications

1. Procédé de formation d'une composition en poudre inhalable comprenant :
la combinaison d'un alcool de sucre avec un alcaloïde et un vecteur liquide pour former un mélange liquide, l'alcool de sucre comprenant de l'érythritol, du myo-inositol, de l'adonitol, du mannitol et du xylitol, ou une combinaison de ceux-ci ;
la lyophilisation du mélange liquide pour former une composition sèche cristalline ; et
le broyage de la composition sèche cristalline pour former des particules de poudre sèche cristalline ayant un diamètre aérodynamique moyen en masse (MMAD) d'environ 5 micromètres ou moins lorsqu'il est mesuré avec un impacteur en cascade.

2. Procédé selon la revendication 1, dans lequel l'étape de combinaison comprend la combinaison de l'alcool de sucre avec un alcaloïde et un acide aminé et un vecteur liquide pour former un mélange liquide.

3. Procédé selon l'une quelconque des revendications précédentes, dans lequel l'étape de broyage comprend le broyage à jet de la composition sèche cristalline pour former des particules de poudre sèche cristalline.

4. Procédé selon l'une quelconque des revendications précédentes, dans lequel l'étape de broyage comprend le broyage de la composition sèche cristalline pour former des particules de poudre sèche cristalline ayant un diamètre aérodynamique moyen en masse (MMAD) dans une plage d'environ 1 micromètre à environ 3 micromètres, ou de 1,5 micromètre à 2,5 micromètres lorsqu'il est mesuré avec un impacteur en cascade.

5. Procédé selon l'une quelconque des revendications précédentes, dans lequel l'alcaloïde comprend un sel d'un alcaloïde.

6. Procédé selon l'une quelconque des revendications précédentes, dans lequel l'alcaloïde comprend du bitartrate de nicotine, ou de l'aspartate de nicotine, du malate de nicotine, ou du glutarate de nicotine.

7. Procédé selon l'une quelconque des revendications précédentes, dans lequel l'alcaloïde comprend un sel d'anatabine.

8. Procédé selon l'une quelconque des revendications précédentes, dans lequel les particules de poudre sèche cristalline comprennent environ 60 % en poids ou plus d'alcool de sucre, et 10 % en poids ou plus d'acide aminé et d'environ 1 % à environ 10 % en poids de sel alcaloïde solide.

9. Procédé selon l'une quelconque des revendications précédentes, dans lequel l'étape de combinaison comprend la combinaison de mannitol, d'érythritol ou de myo-inositol avec un composé alcaloïde et de l'eau pour former un mélange liquide.

10. Procédé selon l'une quelconque des revendications précédentes, dans lequel l'étape de combinaison comprend la combinaison de mannitol, d'érythritol ou de myo-inositol avec un composé nicotinique et de l'eau pour former un mélange liquide.

11. Procédé selon l'une quelconque des revendications précédentes, dans lequel l'étape de combinaison comprend la combinaison de mannitol, d'érythritol ou de myo-inositol avec un composé d'anatabine et de l'eau pour former un mélange liquide.

12. Procédé selon l'une quelconque des revendications précédentes, dans lequel les particules de poudre sèche cristalline présentent une hygroscopicité d'environ 5 % ou moins, ou 4 % ou moins, ou 3 % ou moins, ou 2 % ou moins, ou 1 % ou moins.

13. Procédé selon l'une quelconque des revendications précédentes, dans lequel un acide aminé est ajouté aux particules de poudre sèche cristalline après l'étape de lyophilisation.

14. Procédé selon la revendication 13, dans lequel l'étape de broyage comprend le broyage conjoint des particules de poudre sèche cristalline avec des particules d'acides aminés.

15. Procédé selon la revendication 2, 8, 13 ou 14, dans lequel l'acide aminé comprend de la leucine.
